# EUROPEAN PATENT SPECIFICATION

(11) **EP 1 495 730 B1**
(45) Date of publication and mention of the grant of the patent: **06.12.2006**
(21) Application number: 03015391.0
(22) Date of filing: 08.07.2003
(51) Int. Cl.: A61B 17/70

(54) **Filling device and system for treating a deformed or diseased spine**
Füllbares Implantat und System zur Behandlung einer deformierten oder pathologisch veränderten Wirbelsäule
Dispositif remplissable et système pour le traitement d'une colonne vertébrale déformée ou malade

(43) Date of publication of application: 12.01.2005
(73) Proprietor: A-Spine Holding Group Corp., Road Town, Tortola (VG)
(72) Inventor: Lin, Chih-I, Taipei (TW); Lin, Kwan-Ku, Taipei (TW)
(74) Representative: Grünecker, Kinkeldey, Stockmair & Schwanhäusser Anwaltssozietät

(56) References cited:
- EP-A- 1 132 061
- US-A- 5 549 679
- US-A- 6 017 366
- US-A1- 2002 068 974
- US-A1- 2002 173 796
- US-B1- 6 402 784

## Description

### FIELD OF THE INVENTION

The present invention relates generally to a filling device or system for use in treating a deformed or diseased spine, and more particularly to a flexible and permeable filling device or system which is used to fill in a spinal segment or between two adjacent spinal segments.

### BACKGROUND OF THE INVENTION

The U.S. Pat. Nos. 5,549,679; 5,571,189; 6,375,682B1 disclose respectively various methods for treating a variety of spinal disorders, such as osteoporosis, spinal collapse, etc. In the process of treating such spinal disorders, a trocar is fastened onto a spinal segment so as to form a tubular passageway into which one or more gypsum rods are fitted. The gypsum rods are then forced into the spinal segment. In the course of filling in the spinal segment, the gypsum rods or other artificial bone fillers are bound to scatter aimlessly to inflict harm on the nervous system.

Certain spinal disorders call for implantation of an artificial intervertebral disk, which is administered in conjunction with a balloon made by Kyphon Corporation of the United States. This treatment is disclosed respectively by the U.S. Pat. Nos. 5,927,015; 6,066,154; and 6,248,110B1. The artificial intervertebral disk does not provide a sufficient support and must be therefore augmented by the gypsum filler or other artificial bone filler. The supplementary use of the gypsum filler often results in the aimless dispersion of the gypsum filler, thereby causing injuries to the nervous system. In addition, the artificial intervertebral disk is generally made of a metal material and has a fixed form. As a result, the artificial intervertebral disk is not surgically feasible in terms of the intervertebral support area or support angle.

Intervertebral disc nucleus prosthesis is known from US 6,402,784 B1.

An expandable power mesh back device and methods of use for reduction filling, fixation and supporting of a bone are known from US 2002-0068974 A1.

### SUMMARY OF THE INVENTION

The primary objective of the present invention is to provide a spine filling device comprising a filling member and a pasty medicine. The filling member is first contracted to facilitate the inserting of the filling member and is then forced to regain its original form by the pasty medicine which is injected into the filling member.

A spine filling device according to one aspect of the present invention is given by claim 1. Preferred embodiments are disclosed in the dependent claims.

The pores of the meshed walls of the filling members of the present invention allow the bone cells to grow thereinto to serve as anchors.

The meshed walls of the filling members of the devices of the present invention are made of a biocompatible or biosynthetic material, such as titanium threads, goat intestine threads, or the like.

The meshed walls of the filling members of the devices of the present invention are mixed with metal threads by which the filling members can be precisely located in a spinal segment or between two adjacent spinal segments by a ray imaging system, such as X-ray machine.

If the average diameter of the pores of the meshed walls of the filling members of the present invention is close to 0.1 mm, the walls are preferably formed of two or more layers which are laminated, depending on the viscosity and the particle size of the pasty medicine. For example, if the pasty medicine has a high viscosity or contains larger particles, the walls may be formed of only two layers. On the other hand, if the pasty medicine has a relatively low viscosity or contains smaller particles, the walls are preferably formed of three or more layers.

The flexible and permeable walls of the filling members of the present invention are of a laminated multi-layered construction, the layers are laminated in such a way that the pores of the layers are not aligned.

It must be noted here that the words, such as front, rear, left, and right, are used in this specification. For this reason, the definitions of these words must be clarified. The front refers to a direction in which the device of the present invention is advanced by a surgeon to proceed with the surgical operation. The rear refers to a direction in which the device of the present invention is withdrawn by the surgeon from the surgical operation. The left refers to a direction towards the left arm of the surgeon, while the right refers to a direction towards the right arm of the surgeon.

The features and the advantages of the present invention will be more readily understood upon a thoughtful deliberation of the following detailed description of a preferred embodiment of the present invention with reference to the accompanying drawings.

### BRIEF DESCRIPTION OF THE DRAWINGS

FIG. 1 show a perspective views of the preferred embodiment of the present invention.
FIG. 2 shows an exploded view of the preferred embodiment of the present invention.
FIG. 3a shows a sectional schematic views of one-layered flexible and permeable wall not forming part of the present invention.
FIG. 3b shows a sectional schematic view of multi-layered flexible and permeable wall of the present invention.
FIGS. 4a and 4b are schematic views of the filling members of the preferred embodiment of the present invention at work.
FIGS. 5a-5d are schematic views of the preferred embodiment of the present invention in operation such that the filling member is implanted in a spinal segment.
FIG. 6 shows a schematic view of the implantation of the filling member of the preferred embodiment of the present invention between two adjacent spinal segments.

### DETAILED DESCRIPTION OF THE PREFERRED EMBODIMENTS

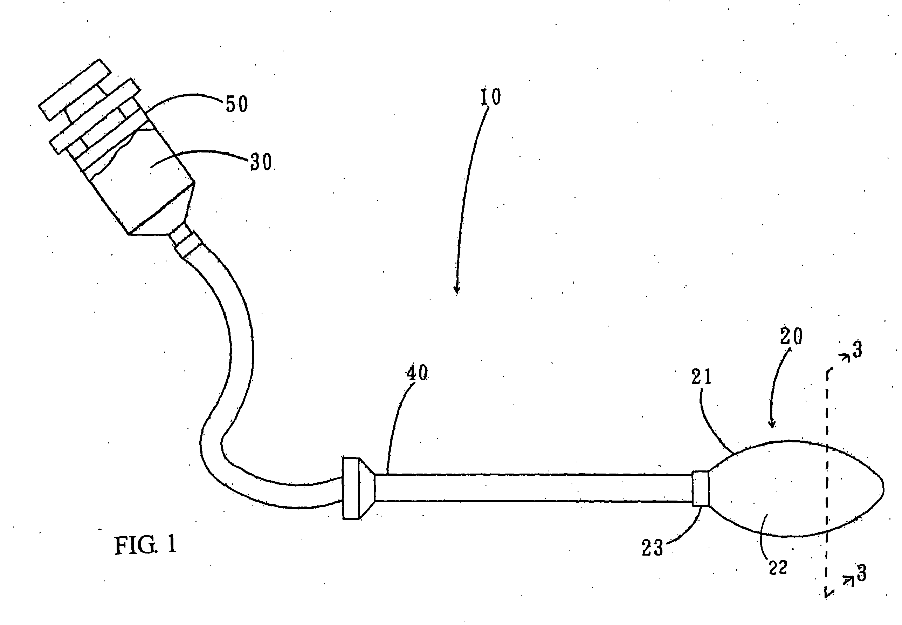

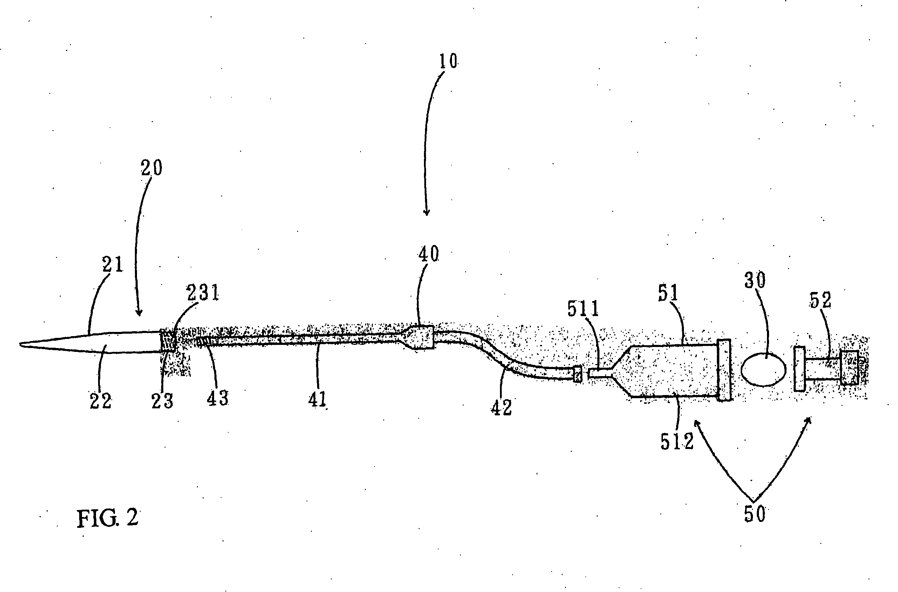

As shown in FIGS. 1 and 2, a spine filling system 10 embodied in the present invention comprises a filling member 20, a pasty medicine 30, a connection tube 40, and a syringe 50.

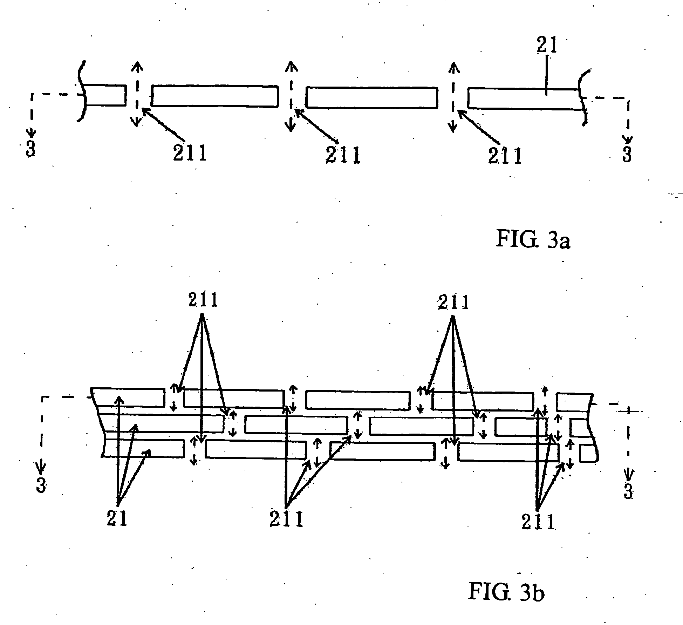

The filling member 20 is made of a flexible and permeable wall 21 and is provided with a holding portion 22 and an injection port 23. The holding portion 22 is confined by the permeable wall 21, which is made by punching or weaving. The injection port 23 is formed at one open end of the filling member 20 by folding and contracting. The injection port 23 is provided with inner threads 231. The wall 21 is made of a biocompatible material or titanium threads and is of a netlike construction. The wall 21 is provided with a plurality of pores 211, as shown in FIGS. 3a and 3b. The pores 211 are formed by computer weaving or laser punching such that the pores 211 have a diameter smaller than 0.1 mm. As a result, the wall 21 is permeable to gas or liquid. However, pores 211 of the wall 21 do not allow passage by solid. The filling member 20 is deformable, as shown in FIGS. 1 and 2.

The pasty medicine 30 is formed of water and one or more kinds of medicines in powdered, or colloidal form. The pasty medicine 30 is capable of solidification. Prior to being injected into the filling member 20, the pasty member 30 is put into the syringe 50, as illustrated in FIG. 2.

The connection tube 40 has one end 41 which is provided with outer threads 43 engageable with the inner threads 231 of the injection port 23 of the filling member 20. As a result, the connection tube 40 can be fastened at one end 41 with the injection port 23 of the filling member 20. the connection tube 40 has another end 42 by which the connected with one end 511 of a barrel 51 of the syringe 50. The barrel 51 is provided with a holding space 512 to accommodate the pasty medicine 30. The syringe 50 further comprises a plunger 52 which is slidably disposed in the holding space 512 of the barrel 51. The pasty medicine 30 is forced by the plunger 52 out of the holding space 512 and into the filling member 20 via the connection tube 40.

As shown in FIG. 3a which is a sectional view taken along a line 3-3 as shown in FIG. 1, the wall 21 of the filling member 20 is of a one-layered construction. The wall 21 may be of a multi-layered construction, as shown in FIG. 3b. There are three layers, which are laminated in such a manner that the pores 211 of the layers are not corresponding in locating, thereby resulting in reduction in permeability of the wall 21.

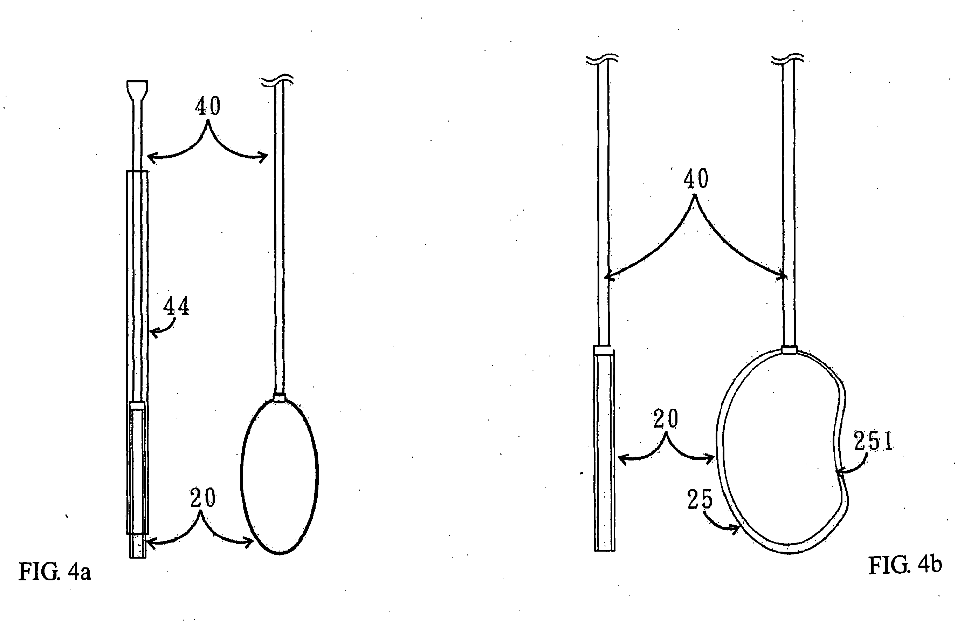

As illustrated in FIGS. 4a and 4b, the filling member is embodied in two patterns. The filling member 20 is originally compressed to have a tubular form 24 and is connected to the connection tube 40. Both the filling member 20 and the connection tube 40 are fitted into a sleeve 44 by which the filling member 20 can be prevented from being deformed or twisted at the time when the filling member 20 is implanted in a spinal (vertebral) segment or between two adjacent vertebrae, as shown in the left side of FIG. 4a. The filling member 20 may take a spherical, oval, oblong, or conical form; nevertheless it can be contracted. As the pasty medicine 30 is injected into the deformed filling member 20, the filling member 20 regains its original form. The right side of FIG. 4a shows that the filling member 20 has regained its original shape upon completion of the injection of the pasty medicine 30 into the filling member 20.

As shown in FIG. 4b, the filling member 20 has a kidney shape 25 and a wall 251. The filling member 20 is intended for use as a substitute for an intervertebral disk in view of the fact that the filling member 20 has a form which is in conformity with the shape of the intervertebral disk. The filling member 20 is made of the titanium threads and is movably connected to the connection tube 40. The filling member 20 is implanted between two adjacent vertebrae without the use of a sleeve which is used to prevent the filling member 20 from being bent.

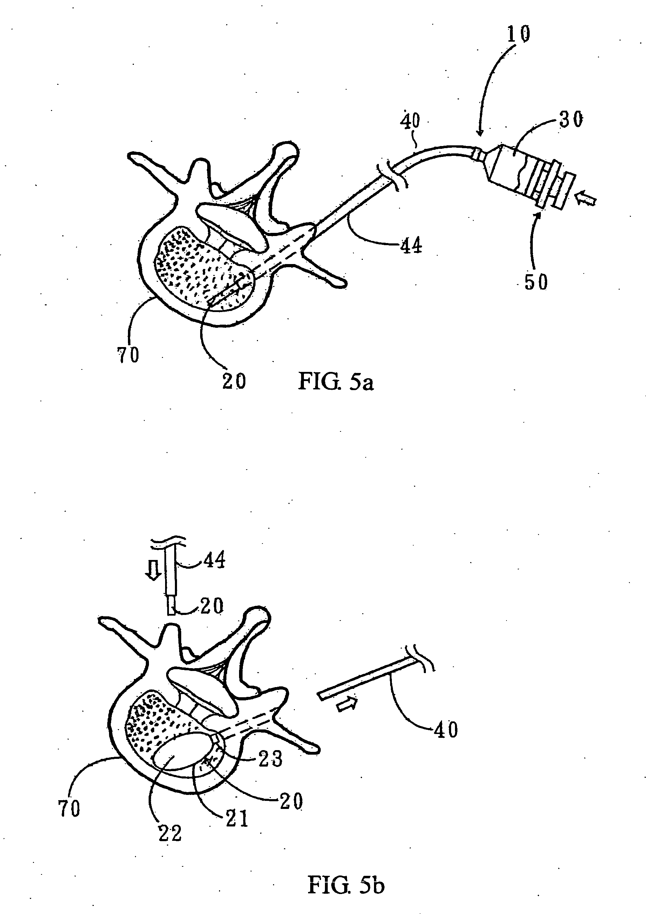

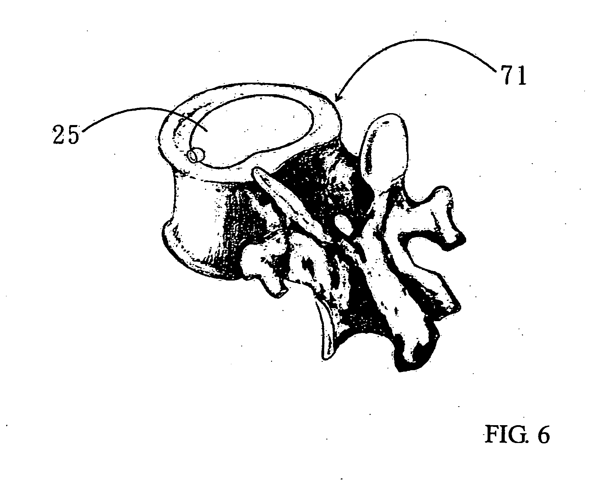

The surgical operation of the spine filling system 10 of the present invention is described hereinafter with reference to FIGS. 5a-5d. The filling member 20 is connected to the connection tube 40. The filling member 20 and the connection tube 40 are fitted into the sleeve 44. With force applied to the connection tube 40 and the sleeve 44, the filling member 20 is inserted into a spinal segment 70 with a small incision. It must be noted here that the filling member 20 may be inserted into the spinal segment 70 without the use of the sleeve 44, depending on the rigidity of a material of which the filling member 20 is made.

The sleeve 44 is gradually moved out from the spinal segment 70. The pasty medicine 30 is forced into the filling member 20 by the syringe 50 in conjunction with the connection tube 40. The pasty medicine 30 puts a pressure on the wall 21 of the filling member 20, so as to cause the filling member 20 to regain its original form. In the meantime, a portion of the pasty medicine 30 permeates through the wall 21 to the outside of the filling member 20.

Upon completion of solidification of the pasty medicine 30, the connection tube 40 is disconnected with the filling member 20. The pasty medicine 30 serves to give an added support to the spinal segment 70. The pasty medicine 30 may contain poly(methylmethacrylate), hydroxy apatite, etc. In order to reinforce the support of the spinal segment 70, a left filling member 20 may be implanted side by side to the filling member 20 at the right side in the spinal segment 70, as shown in FIG. 5b.

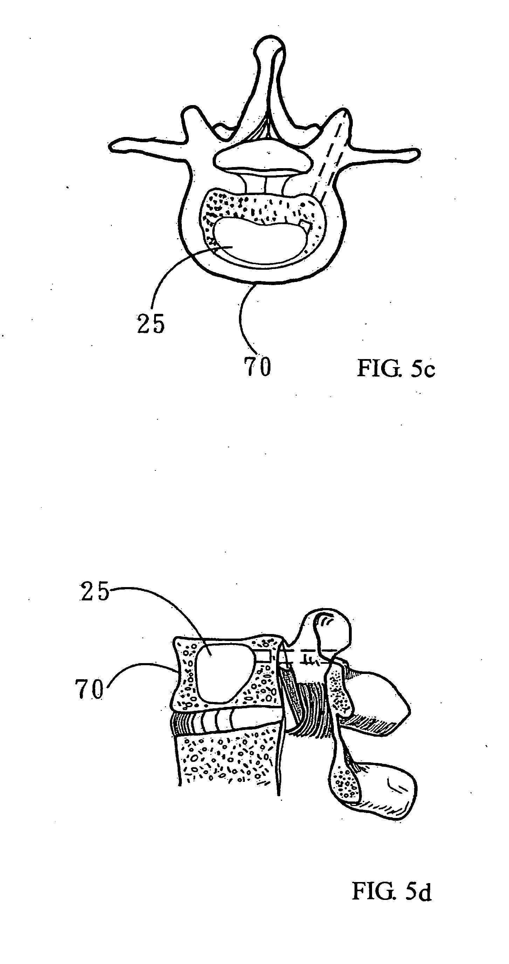

As illustrated in FIG. 5c, a kidney-shaped filling member 25 is implanted in the spinal segment 70 which has collapsed. FIG. 5d shows a sectional view of the spinal segment 70 in which the filling member 25 is implanted.

As shown in FIG. 6, the kidney-shaped filling member 25 is implanted in an intervertebral space 71. The filling member 25 contains the pasty medicine 30 and serves as an artificial intervertebral disk. The filling member 25 is shaped like kidney to conform to the shape of the intervertebral disk.

## Claims

1. A spine filling device (10) comprising:
a flexible and permeable filling member (20) formed of one or more meshed walls (21) and provided with a holding portion (22) and an injection port (23) in communication with said holding portion (22), said meshed walls (27) being elastically compressible and being a laminated multi-layer wall, and each layer of which is provided with a plurality of pores (211), each having a diameter smaller than 0.1 mm, so that said meshed walls (21) are not airtight; and
a pasty medicine (30) capable of solidification to be injected into said holding portion (22) via said injection port (23) of said filling member (20),
wherein said injection of said pasty medicine (30) into said holding portion (22) results in expansion of said filling member (20), and said filing member (20) is to be implanted in a vertebral segment (70) or an intervertebral space, whereby said filling member is to be securely lodged in the vertebral segment (70) or the intervertebral space upon completion of the solidification of said pasty medicine (30), **characterized in that** the layers of the laminated multi-layer wall are laminated in such a way that the pores (211) of the layers are not aligned, and that the laminated multi-layer wall is permeable to liquid and the laminated multi-layer wall do not allow passage by solid.

2. The spine filling device as defined in claim 1, wherein said filling member (20) takes the form of a sac, bag, or ball.

3. The spine filling device as defined in claim 1, wherein said pasty medicine (30) is a mixture of a liquid and a bone cement powder.

4. The spine filling device as defined in claim 3, wherein said bone cement powder is selected from the group consisting of gypsum, calcium sulfate, calcium phosphate, poly(methylmethacrylate) and hydroxy apatite.

5. The spine filling device as defined in claim 1 further comprising an injection tool (40, 50) to be fastened detachably with said filling member (20), so that said pasty medicine (30) is to be injected into said holding portion (22) via said injection tool.

6. The spine filling device as defined in claim 5, wherein said injection tool comprises' a connection tube (40) and a syringe (50) formed of a barrel and a plunger (52), wherein said connection tube (40) is to be fastened detachably at one end with said injection port (22) of said filling member (20), said connection tube (40) is further to be fastened at other end with said barrel (51) of said syringe (50); wherein said pasty medicine (30) is to be injected into said holding portion (22) of said filling member (20) from said barrel (51) by said plunger (52)via said connection tube (40).

## Patentansprüche

1. Wirbelsäulen-Füllvorrichtung (10), die umfasst:
ein flexibles und durchlässiges Füllelement (20), das aus einer oder mehreren Maschenwänden (21) besteht und mit einem Aufnahmeabschnitt (22) sowie einem Einspritzanschluss (23) versehen ist, der mit dem Halteabschnitt (22) in Verbindung steht, wobei die Maschenwände (27) elastisch zusammengedrückt werden können und eine laminierte mehrschichtige Wand sind und jede Schicht derselben mit einer Vielzahl von Poren (211) versehen ist, die jeweils einen Durchmesser von weniger als 0,1 mm haben, so dass die Maschenwände (21) nicht luftdicht sind; und
ein pastöses Medikament (30), das sich verfestigen kann und über den Einspritzanschluss (23) des Füllelementes (20) in den Aufnahmeabschnitt (22) einzuspritzen ist,
wobei das Einspritzen des pastösen Medikaments (30) in den Aufnahmeabschnitt (22) zur Ausdehnung des Füllelementes (20) führt, das Füllelement (20) in ein Wirbelsegment (70) oder einen Zwischenwirbelraum zu implantieren ist und das Füllelement nach Abschluss der Verfestigung des pastösen Medikaments (30) fest in dem Wirbelsegment (70) oder dem Zwischenwirbelraum aufgenommen sein soll, **dadurch gekennzeichnet, dass** die Schichten der laminierten mehrschichtigen Wand so laminiert sind, dass die Poren (211) der Schichten nicht fluchtend sind, und dass die laminierte mehrschichtige Wand durchlässig für Flüssigkeit ist und die laminierte mehrschichtige Wand das Hindurchtreten von Feststoffen nicht zulässt.

2. Wirbelsäulen-Füllvorrichtung nach Anspruch 1, wobei das Füllelement (20) die Form eines Sacks, eines Beutels oder einer Kugel hat.

3. Wirbelsäulen-Füllvorrichtung nach Anspruch 1, wobei das pastöse Medikament (30) ein Gemisch aus einer Flüssigkeit und einem Knochenzementpulver ist.

4. Wirbelsäulen-Füllvorrichtung nach Anspruch 3, wobei das Knochenzementpulver aus der Gruppe ausgewählt wird, die aus Gips, Kalziumsulfat, Kalziumphosphat, Poly(methylmethacrylat) und Hydroxyapatit besteht.

5. Wirbelsäulen-Füllvorrichtung nach Anspruch 1, die des Weiteren ein Einspritzwerkzeug (40, 50) umfasst, das abnehmbar an dem Füllelement (20) zu befestigen ist, so dass das pastöse Medikament (30) über das Einspritzwerkzeug in den Aufnahmeabschnitt (22) einzuspritzen ist.

6. Wirbelsäulen-Füllvorrichtung nach Anspruch 5, wobei das Einspritzwerkzeug eine Verbindungsröhre (40) und eine Spritze (50) umfasst, die aus einem Zylinder und einem Kolben (52) besteht, die Verbindungsröhre (40) abnehmbar an einem Ende an dem Einspritzanschluss (22) des Füllelementes (20) zu befestigen ist, die Verbindungsröhre (40) des Weiteren an dem anderen Ende an dem Zylinder (51) der Spritze (50) zu befestigen ist und das pastöse Medikament (30) durch den Kolben (52) über die Verbindungsröhre (40) aus dem Zylinder (51) in den Aufnahmeabschnitt (22) des Füllelementes (20) einzuspritzen ist.

## Revendications

1. Dispositif de remplissage de colonne vertébrale (10) comprenant :
un élément de remplissage souple et perméable (20) formé d'une ou de plusieurs parois maillées (21) et comportant une partie de maintien (22) et un orifice d'injection (23) en communication avec ladite partie de maintien (22), lesdites parois maillées (27) étant compressibles élastiquement et constituant une paroi multicouche stratifiée dont chaque couche est munie de plusieurs pores (211) ayant chacun un diamètre inférieur à 0,1 mm afin que lesdites parois maillées (21) ne soient pas étanches à l'air ; et
un médicament pâteux (30) capable de se solidifier et destiné à être injecté dans ladite partie de maintien (22) par l'intermédiaire dudit orifice d'injection (23) dudit élément de remplissage (20),
ladite injection dudit médicament pâteux (30) dans ladite partie de maintien (22) provoquant l'expansion dudit élément de remplissage (20), lequel est destiné à être implanté dans un segment vertébral (70) ou dans un espace intervertébral pour être ainsi logé de manière fixe dans le segment vertébral (70) ou dans l'espace intervertébral à la fin de la solidification dudit médicament pâteux (30), **caractérisé en ce que** les couches de la paroi multicouche stratifiée sont superposées de façon que leurs pores (211) ne soient pas alignés, et **en ce que** la paroi multicouche stratifiée est perméable aux liquides et ne permet pas le passage de solides.

2. Dispositif de remplissage de colonne vertébrale selon la revendication 1, dans lequel ledit élément de remplissage (20) prend la forme d'un sac, d'une poche ou d'un ballon.

3. Dispositif de remplissage de colonne vertébrale selon la revendication 1, dans lequel ledit médicament pâteux (30) est un mélange d'un liquide et d'une poudre de ciment osseux.

4. Dispositif de remplissage de colonne vertébrale selon la revendication 3, dans lequel ladite poudre de ciment osseux est sélectionnée dans le groupe constitué par le gypse, le sulfate de calcium, le phosphate de calcium, le poly(méthacrylate de méthyle) et l'hydroxy apatite.

5. Dispositif de remplissage de colonne vertébrale selon la revendication 1, comprenant en outre un outil d'injection (40, 50) destiné à être fixé de manière amovible audit élément de remplissage (20) afin que le médicament pâteux (30) puisse être injecté dans ladite partie de maintien (22) par l'intermédiaire dudit outil d'injection.

6. Dispositif de remplissage de colonne vertébrale selon la revendication 5, dans lequel ledit outil d'injection comprend un tube de raccordement (40) et une seringue (50) constituée d'un cylindre et d'un piston (52), dans lequel ledit tube de raccordement (40) est destiné à être fixé de manière amovible au niveau de l'une de ses extrémités audit orifice d'injection (22) dudit élément de remplissage (20), ledit tube de raccordement (40) étant également destiné à être fixé au niveau de son autre extrémité audit cylindre (51) de ladite seringue (50) ; dans lequel ledit médicament pâteux (30) est destiné à être injecté dans ladite partie de maintien (22) dudit élément de remplissage (20) à partir dudit cylindre (51) par ledit piston (52), par l'intermédiaire dudit tube de raccordement (40).
